# EUROPEAN PATENT APPLICATION

(11) **EP 2 808 887 A1**
(43) Date of publication of application: **03.12.2014**
(21) Application number: 14170358.7
(22) Date of filing: 28.05.2014
(51) Int. Cl.: H01J 49/04, G01N 33/68

(54) **Measurement plate for MALDI mass spectrometry**

(30) Priority: 29.05.2013 JP 2013113447
(71) Applicant: Shimadzu Corporation, Kyoto 604-8511 (JP); Panasonic Corporation, Osaka 571-8501 (JP)
(72) Inventor: Shimada, Takashi, Kyoto, Kyoto 604-8511 (JP); Aoki, Chikage, Kyoto, Kyoto 604-8511 (JP); Sato, Taka-Aki, Kyoto, Kyoto 604-8511 (JP); Nakatani, Masaya, Osaka 540-6207 (JP); Hayama, Masaaki, Osaka 540-6207 (JP)
(74) Representative: Webster, Jeremy Mark

(57) **Abstract**

A measurement plate 10 for MALDI mass spectrometry consists of a base plate 11 having an upper surface and a carbon sheet 12 laid on at least a portion of the upper surface. The carbon sheet 12 is a highly-oriented graphite sheet with the orientation direction being parallel to the upper surface. The highly-oriented graphite sheet has a high thermal conductivity in the in-plane direction and a low thermal conductivity in the perpendicular direction. Therefore, the thermal energy generated in a matrix substance by laser irradiation is quickly transferred through the highly-oriented graphite sheet in the in-plane direction. As a result, the thermal energy is uniformly distributed within the laser-irradiated area, so that the reaction of sublimation and ionization of the target sample assisted by the matrix substance uniformly takes place. Thus, a decrease in the detection sensitivity is prevented. Optionally, a high electrical conductivity plate is inserted in the electrically conductive adhesive layer fixing the base plate and the graphite sheet.

## Description

### TECHNICAL FIELD

The present invention relates to a device used in an ionizing section where a target substance is ionized for performing a mass spectrometry in a matrix-assisted laser desorption/ionization (MALDI) mass spectrometer which is widely used for measuring the masses of the molecules of proteins, peptides, organic compounds or similar samples. In particular, it relates to a sample plate (measurement plate) used in the ionizing section.

### BACKGROUND ART

The MALDI mass spectrometry is a technique capable of ionizing proteins (whose molecular weight is normally 10000 or greater), peptides (whose molecular weight is normally 1000 or greater) or other substances with comparatively high molecular weights without causing unwanted breakage of the substance. Due to this feature, the technique has been widely used for analyzing those kinds of substances. It is believed that, in the MALDI process, the laser ionization of the target substance occurs without breaking the target substance since the optical energy of the laser is not directly supplied to the target substance; the optical energy is initially converted into thermal energy by the matrix substance which surrounds the target substance, and this thermal energy is used as the ionization energy for the target substance.

The amount of target substance that can be correctly (i.e. without breakage) ionized (or the ionization efficiency) depends on the kind of target substance, the kind of matrix substance and other factors. Therefore, for each target substance, it is necessary to select a matrix substance and optimize measurement conditions, such as the laser irradiation conditions and the sample preparation conditions.

Thus, the devices used in the ionizing section where a target substance is ionized are extremely important in the MALDI mass spectrometer.

For the detection of a target substance that is contained at a low concentration, various methods have been conventionally proposed. For example, the detection limit of the substance concentration has been conventionally at a level of approximately 1 fmol/µL (femtomol per microliter). To detect substances at concentrations lower than this limit, a method has been developed in which the measurement sensitivity is improved by condensing the target substance and putting the obtained dense target substance on a measurement plate. One example is disclosed in Non-Patent Literature 1, in which the measurement plate made of stainless steel (SUS), with the surface area divided into hydrophobic and hydrophilic regions, is used as the sample plate on which the matrix substance and the target compound are to be adsorbed. A liquid sample is dropped onto this measurement plate. As the sample dries, the target substance becomes condensed on the hydrophilic region. Thus, even if the initial concentration of the target substance in the liquid sample is low, the target substance is densely contained in the sample after the drying process and hence can be easily detected in the measurement.

Patent Literature 1 discloses an example in which a specific kind of protein is selectively captured on the measurement plate to efficiently detect this protein.
Patent Literature 2 discloses the fact that forming extremely small projections (quantum dots) on the surface of the measurement plate is effective for improving the ionization efficiency.

### CITATION LIST

### PATENT LITERATURE

Patent Literature 1: US 2003-0173513 A
Patent Literature 2: JP 2006-329977 A
Patent Literature 3: JP 1-018002 B
Patent Literature 4: JP 7-023261 B

### NON PATENT LITERATURE

Non Patent Literature 1: "µFocus Sample Plates for MALDI-TOF MS", published on the website of Hudson Surface Technology, Inc. at http://www.maldiplate.com/sites/default/files/assets/uFocus%20Technology%202010-08-23%20hires.pdf, as searched on April 10, 2013

Non Patent Literature 2: N. Nishiki et al., "Juunansei Wo Yuusuru Kesshousei Gurafaito No Kaihatsu To Jitsuyouka (Development and Practical Application of Flexible Crystalline Graphite)", published on the website of Panasonic Corporation at http://panasonic.co.jp/ptj/award/2010/pdf/42nd_ichimura_01.pdf, as searched on April 10, 2013

### SUMMARY OF INVENTION

### THECHNICAL PROBLEM

In the method described in Non-Patent Literature 1, it is assumed that the condensation of the liquid sample uniformly takes place during the drying process. However, the condensation may possibly occur non-uniformly depending on the drying conditions or the physicochemical natures of the target substance or the matrix. In the methods described in Patent Literatures 1 and 2, if the concentration of the matrix in the region surrounding the target substance is non-uniform, the detection accuracy of the target substance will be low, since the method relies on the transfer of thermal energy from the matrix substance to the target substance.

The problem to be solved by the present invention is to provide a system and a method in which the previously described decrease in the detection accuracy due to the non-uniformity in the distribution of the target substance or matrix is minimized.

### SOLUTION TO PROBLEM

A measurement plate for MALDI mass spectrometry according to the present invention aimed at solving the previously described problem includes:
a base plate having an upper surface; and
a highly-oriented graphite sheet laid on at least a portion of the upper surface, with the orientation direction of the graphite sheet being parallel to the upper surface.

### ADVANTAGEOUS EFFECTS OF THE INVENTION

Due to the previously described structure, the measurement plate for MALDI mass spectrometry according to the present invention has a higher thermal conductivity in the in-plane direction of its upper surface and a lower thermal conductivity in the perpendicular direction (i.e. in the depth direction of the plate). That is to say, the plate is anisotropic in terms of thermal conductivity. Due to this property, the thermal energy generated in the matrix substance by laser irradiation is quickly transferred through the highly-oriented graphite sheet in the in-plane direction. As a result, the thermal energy is uniformly distributed within the laser-irradiated area, so that the reactions of sublimation and ionization of the target sample assisted by the matrix substance uniformly takes place. Thus, the decrease in the detection sensitivity is prevented. Various other effects can also be obtained, such as the capability of high-sensitivity detection that does not significantly depend on the state of the base plate, as will be described later.

### BRIEF DESCRIPTION OF DRAWINGS

Figs. 1A and 1B are a schematic perspective view and a sectional view of a measurement plate for mass spectrometry as one embodiment of the present invention, respectively.
Figs. 2A and 2B are enlarged views respectively showing an upper surface and a section of a highly-oriented graphite sheet.
Fig. 3 is an explanatory diagram showing a difference between the thermal conductivity in the in-plane (horizontal) direction and the thermal conductivity in the out-of-plane (vertical) thermal conductivity of a highly-oriented graphite sheet.
Fig. 4 is a schematic perspective view of a measurement plate for mass spectrometry as another embodiment of the present invention.
Fig. 5 is a graph comparing the amount of ions produced using the measurement plate of the first embodiment of the present invention and that of the ions produced using a conventional measurement plate.
Fig. 6 is a graph showing the effect produced by inserting a thin plate having a high electrical conductivity between the base plate and the highly-oriented graphite sheet.
Fig. 7 is a graph showing the effect produced by adhering a highly-oriented graphite sheet in the case where the base plate has holes formed on its reverse side.
Figs. 8A and 8B are mass spectra obtained by analyzing ACTH with a MALDI mass spectrometer, where Fig. 8A shows the result of a measurement in which the target sample was placed on a conventional SUS base plate, and Fig. 8B shows the result of a measurement in which a highly-oriented graphite sheet was adhered to the same SUS base plate and the target sample was placed on that sheet.

### DESCRIPTION OF EMBODMENTS

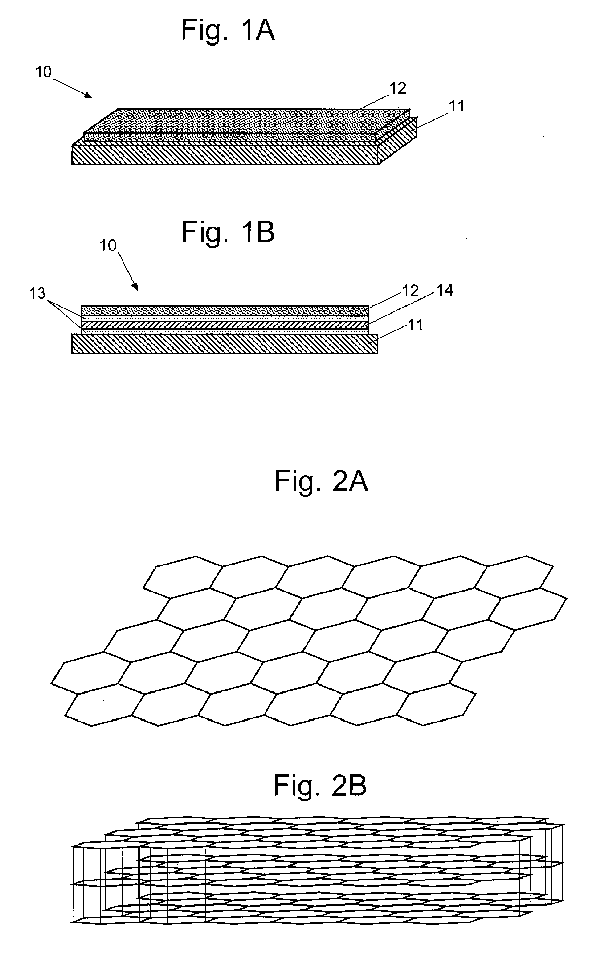

One embodiment of the measurement plate for mass spectrometry according to the present invention is hereinafter described. Fig. 1A is a perspective view of the measurement plate for mass spectrometry according to the present embodiment, and Fig. 1B is a sectional view of the same plate. It should be noted that Figs. 1A and 1B have been drawn for ease of understanding and do not show the real shape, real detailed structure, nor in particular, real dimensional ratio of the plate.

The measurement plate 10 for mass spectrometry of the present embodiment basically consists of a base plate 11 having an upper surface and a carbon sheet 12 laid on the upper surface of the base plate 11. The base plate 11 should preferably be made of an electrically conductive material, such as stainless steel (SUS), copper (Cu) or aluminum (Al), so as to apply an ion-releasing voltage between the base plate 11 and an electrode opposing this plate. Stainless steel (SUS) is the most appropriate material in view of the thermal conductivity, heat resistance, as well as resistance against chemical reactions with solvents or against thermal oxidation. The base plate 11 itself does not always need to have electrical conductivity. For example, the ion-releasing voltage can be applied between the carbon sheet 12 and the opposing electrode.

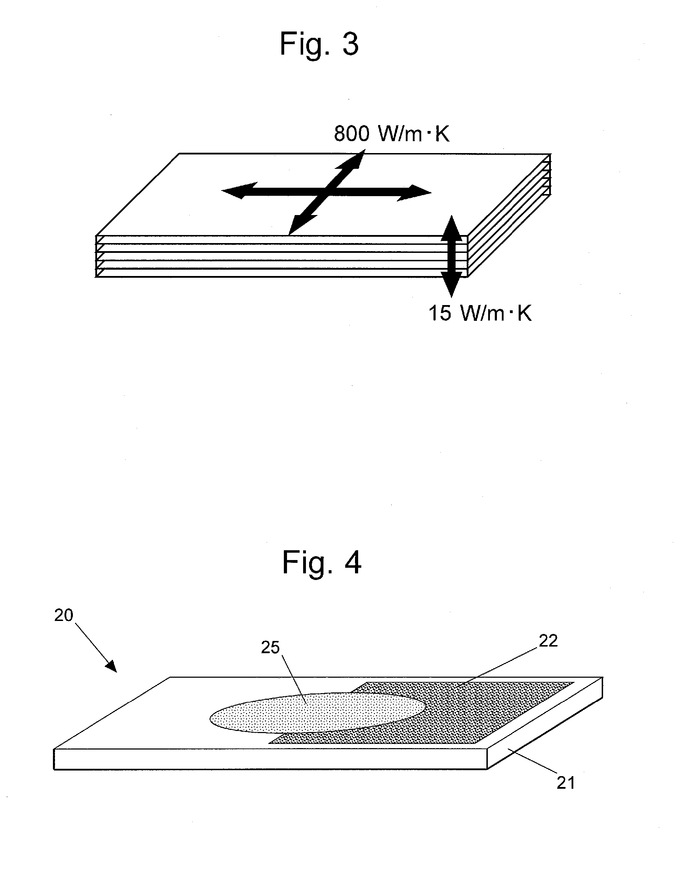

The carbon sheet 12 is made of a highly-oriented graphite sheet. Its orientation direction is aligned with a direction parallel to the upper surface of the base plate 11. As shown in Fig. 2A, the highly-oriented graphite sheet has a layered structure, with each layer having carbon atoms strongly bonded to each other to form a crystalline graphite structure in the horizontal direction. By contrast, in the direction perpendicular to those layers (vertical direction), the bond is created by a mere Van der Waals force, as shown in Fig. 2B. Such a highly-oriented structure with the crystalline structures aligned in the horizontal direction has an extremely high level of thermal conductivity in the horizontal direction, as shown in Fig. 3. By contrast, the thermal conductivity in the vertical direction is low. The ratio of the former thermal conductivity to the latter is greater than 50.

Such a highly-oriented graphite sheet can be produced as follows (for example, see Patent Literature 3 or 4, or Non-Patent Literature 2): Hydrogen, oxygen and nitrogen are desorbed from a specific kind of polymer (e.g. polyimide) by heating it to 3000 degrees Celsius under an oxygen-free condition, after which the remaining carbon atoms are crystalized by annealing. The obtained product has a layer structure, as described earlier.

The carbon sheet 12 is adhered to the upper surface of the base plate 11 with an electrically conductive adhesive 13. A thin plate 14 having a high electrical conductivity should preferably be inserted in the electrically conductive adhesive layer 13. This design ensures a more reliable application of an ion-accelerating voltage to the target sample on the carbon sheet 12. For example, inserting a high electrical conductivity plate 14 made of silver (Ag) into an electrically conductive adhesive layer 13 of approximately 10 µm in thickness lowers the electrical resistance between the base plate 11 and the carbon sheet 12 to roughly 100 Ω or lower. Copper (Cu), aluminum (Al), platinum (Pt) and other kinds of metal may also be used for the high electrical conductivity plate 14 instead of silver (Ag). Non-metallic materials having high electrical conductivity may also be used. It should be noted that the high electrical conductivity plate 14 is not indispensable for the present invention and may be omitted.

The effect of the measurement plate for mass spectrometry of the present embodiment is hereinafter described.

The measurement plate of the present embodiment is designed for use in a mass spectrometer in which a target sample is placed on a measurement plate and irradiated with a laser to ionize a portion of the sample with the thermal energy generated by the laser. Its basic mechanism is widely known as the matrix-assisted laser desorption/ionization mass spectrometry (MALDI-MS) or soft laser desorption/ionization mass spectrometry. The basic procedure is as follows.

A sample containing a substance to be analyzed (detected) is mixed in a predetermined kind of matrix substance (this mixture is called the target sample). A cationic or anionic substance may also be added as needed. In order to uniformly mix the matrix, cationic, anionic and other substances in the target sample, these substances may be dissolved in a solvent.

Subsequently, a predetermined quantity of the target sample thus mixed is put on a designated area on the carbon sheet of the measurement plate. In the case where the target sample is dissolved in a solvent, a waiting time is provided to vaporize the solvent, leaving a deposit of the sample, matrix and other substances.

After the target sample is thus put on the designated area on the carbon sheet of the measurement plate, the measurement plate is set on a designated sample stage in the mass spectrometer.

According to a program prepared for the mass spectrometer, a laser beam is cast onto a predetermined spot on the measurement plate on the sample stage to generate ions from the target sample. Due to the acceleration voltage applied to the measurement plate, the generated ions are released from the measurement plate and accelerated. After flying a predetermined distance within the space between the electrodes provided in the device, the ions are detected by a detector. By measuring the time of flight (TOF) of each ion, the masses of the generated ions can be determined. It should be noted that the masses of the ions can be measured by various methods other than the time-of-flight measurement.

When the target sample is irradiated with the laser, the laser energy is converted into thermal energy in the matrix substance, causing an increase in the temperature of the irradiated spot. During this heat-up process, the target sample sublimates from a solid into a gas. Concurrently, a portion of H⁺ ions (i.e. protons) possessed by the matrix substance are transferred to the sample, whereby the sample is ionized. This reaction is driven by the thermal energy resulting from the energy conversion in the matrix substance. Accordingly, if the thermal energy resulting from the energy conversion in the matrix substance is accumulated in a limited region, the distribution of the thermal energy will be non-uniform, which may possibly result in a non-uniformity in the ionization efficiency. To prevent this situation, the thermal energy resulting from the energy conversion in the matrix substance should be quickly transferred to the surrounding region without being accumulated in a limited region.

The previous example was the case where H⁺ ions are transferred from the matrix substance to the sample. In the case where a cationic or anionic substance is added, the ions received from this substance are used for ionizing the sample. This reaction is also driven by the thermal energy. Therefore, the thermal energy distribution in the target sample is once again significantly important.

It should be noted that the previously described operational procedure of the mass spectrometer is a specific example based on the MALDI-TOF method and does not limit the range of devices and methods in which the present invention is applicable.

As already explained, the measurement plate for mass spectrometry according to the present invention has an anisotropic structure with the thermal conductivity in the in-plane direction (horizontal direction) significantly higher than the thermal conductivity in the out-of-plane direction (depth or vertical direction). Due to this anisotropy, the thermal energy generated by laser irradiation is quickly and preferentially transferred in the horizontal direction. As a result, a uniform energy distribution is achieved, so that the sublimation and ionization of the sample through the matrix substance occurs uniformly.

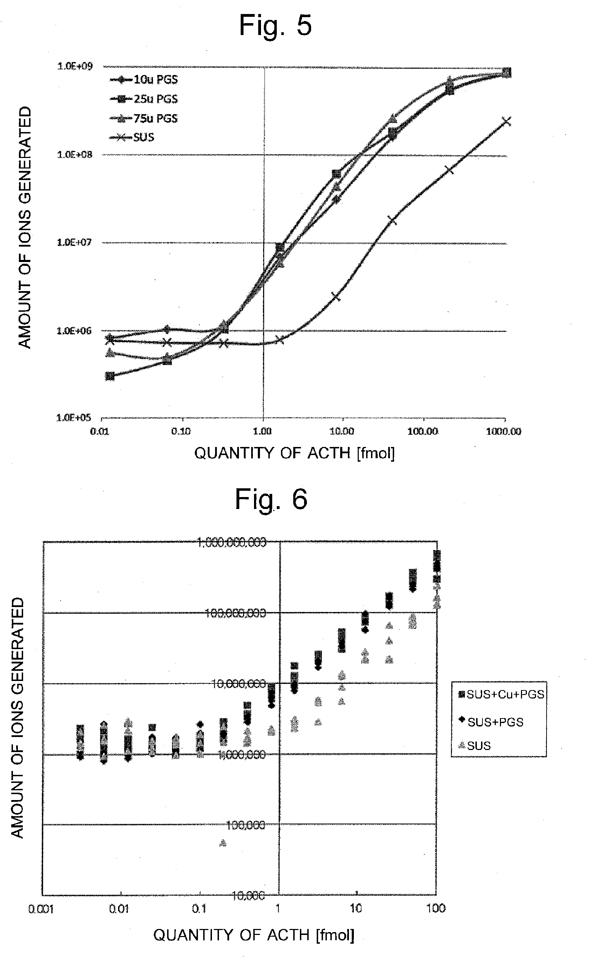

A test for comparing the performance of the measurement plate for mass spectrometry according to the present invention with that of a conventional measurement plate was conducted. The test result is hereinafter described with reference to Fig. 5.

In the test, the amount of ions generated by MALDI was measured with a mass spectrometer for each of the four measurement plates, one of which was a conventional measurement plate (0.5 mm thick) made of pure SUS while the other measurement plates were prepared according to the present invention by laying a highly-oriented graphite sheet (labeled "PGS" in Fig. 5) with a thickness of 10 µm, 25 µm or 75 µm on the aforementioned SUS plate serving as the base plate. A high-vacuum grease marketed under the trade name of "Apiezon" by Edwards Limited, an American company, was used to adhere the highly-oriented graphite sheet to the base plate.

The ionization conditions used in the test of Fig. 5 were as follows:
Sample: ACTH peptide fragment (m/z=2465.20)
Sample Quantities: Two-fold serial dilutions starting from 1 pmol/well (1 pmol in one sample-receiving well on a measurement plate) were prepared (i.e. 1 pmol, 500 fmol, 250 fmol, 125 fmol, 63 fmol, 31 fmol, 16 fmol, 8 fmol, 4 fmol, 2 fmol, 1 fmol, 500 amol, 250 amol, 125 amol, 63 amol, 31 amol, 16 amol, 8 amol, 4 amol, 2 amol and 1 amol/well)
MALDI Matrix: 5 mg/ml CHCA(0.1% TFA + 50% MeCN)
Mass Spectrometer: AXIMA CFRplus MALDI-TOF MS (manufactured by Shimadzu Corporation)
Measurement
Measurement Range: m/z 1400-5000
Analysis Range on MALDI Target: 1×1 mm, 50 µm/point, 441 points
Laser Wavelength: 337 nm (UV laser)
Laser Irradiation Conditions: Output level 70 (in relative value), two shots per point Pulsed Extraction: m/z 4000
ACTH: adrenocorticotropic hormone
CHCA: alpha-cyano-4-hydroxycinnamic acid
TFA: trifluoroacetic acid
MeCN: acetonitrile

In Fig. 5, the measurement limit was approximately 1.6 fmol/well when the conventional measurement plate made of SUS was used, while the limit was improved to approximately 0.32 amol/well when any of the highly-oriented graphite sheets was inserted between the target sample and the SUS base plate. Thus, it has been revealed that the sensitivity of MALDI mass spectrometers can be dramatically improved by using a measurement plate according to the present invention.

Fig. 6 shows the result of another test performed for investigating an effect produced by inserting a thin plate having high electrical conductivity between the SUS base plate and the highly-oriented graphite sheet. As shown in Fig. 6, when a copper plate of approximately 9 µm in thickness was inserted between the SUS base plate and the highly-oriented graphite sheet, a greater amount of ions were generated from almost all the samples with ACTH quantities of 0.001-100 fmol than in the case where the copper plate (high electrical conductivity plate) was not inserted. It is also evident from Fig. 6 that, as in the case of Fig. 5, a measurement plate with a highly-oriented graphite sheet can produce a greater amount of ions than a measurement plate made of a simple SUS plate.

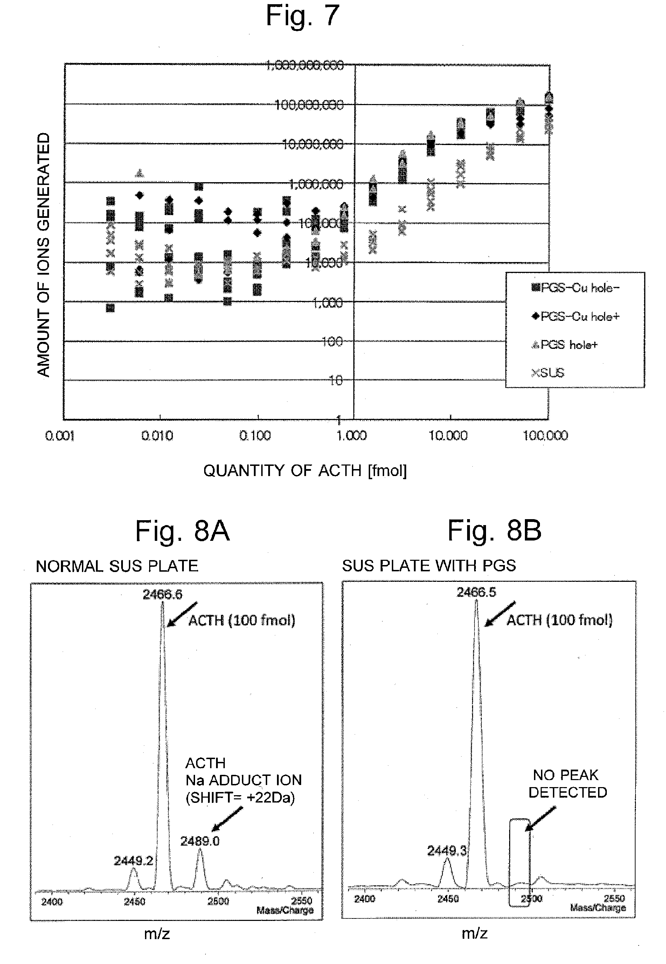

Another test was performed to demonstrate that the measurement plate for mass spectrometry according to the present invention is more robust against disturbances than the conventional measurement plate. The test result is hereinafter described with reference to Fig. 7, which shows the amount of generated ions measured for each of the sample quantities using the following measurement plates: the aforementioned conventional SUS base plate ("SUS"); a measurement plate with a carbon sheet adhered to a SUS base plate with holes formed on its reverse side ("PGS hole+"); a measurement plate prepared by inserting a copper plate in the adhesive layer of the PGS hole+ plate ("PGS-Cu hole+"); and a measurement plate prepared by laying the copper plate and the carbon sheet on the SUS base plate with no holes ("PGS-Cu hole-"). All the plates with the carbon sheet achieved high levels of ion generation efficiencies regardless of whether or not holes were present on the reverse side of the base plate. Conventional measurement plates for mass spectrometry have the problem that a presence of deformation, scratches or other defects in the measurement plate distorts the electric field created by the voltage applied between the base plate and the opposing electrode, which prevents ions from being correctly released from the measurement plate. The test result demonstrates that the measurement plate with the highly-oriented graphite sheet adhered according to the present invention causes ionization in a desirable form regardless of the condition of the base plate. This is also due to the capability of the highly-oriented graphite sheet to efficiently transfer the incident laser energy from the irradiated spots on the target sample through the entire area of this sample, with the favorable result that a high level of ionization efficiency is achieved over the entire area without being significantly affected by the condition of the base plate beneath the highly-oriented graphite sheet.

The highly-oriented graphite sheet should preferably have a surface roughness of 10 nm to 1 µm. The presence of such surface roughness promotes the supply of ions (protons, cations or the like) to the sample.

Fig. 8 shows a test result demonstrating another effect of the measurement plate for mass spectrometry according to the present invention. Normally, a biological molecule is often detected as a peak of a metal adduct ion. However, for many biological molecules, the presence of such a peak of the metal adduct ion deteriorates the measurement sensitivity of the molecule itself. Figs. 8A and 8B show the results of measurements performed for ACTH using a MALDI mass spectrometer. Specifically, Fig. 8A is the result of a measurement in which the target sample was put on a conventional SUS base plate, while Fig. 8B is the result of a measurement in which a highly-oriented graphite sheet was adhered to the same SUS base plate and the target sample was placed on that sheet. The two figures show that, although ACTH has a metal adduct ion (Fig. 8A), the corresponding peak is not present in Fig. 8B. This is probably because the improved heat-flow efficiency due to the highly-oriented graphite sheet significantly promotes the detection of the protonated ion and consequently decreases the relative intensity of the metal adduct ion, thus contributing to the improvement of the measurement sensitivity for the target sample.

As described thus far, even without preparing a high-precision SUS plate, the sensitivity of the MALDI mass spectrometric measurement can be dramatically improved by using a highly-oriented graphite sheet. A significant decrease in the material cost can also be expected from this design.

The measurement plate for mass spectrometry according to the present invention is also expected to produce significant effects in an imaging mass analysis. In the imaging mass analysis, a mass spectrometry is performed for each of the micro areas on a sample, and the analysis results obtained at the respective micro areas are shown on a sample image, with each result presented at the corresponding position on the sample. In this analysis, it is difficult to achieve high sensitivity since there is an abundance of impurities impeding the ionization. It can be expected that using the measurement plate for mass spectrometry according to the present invention significantly raises the sensitivity level and enables a high-accuracy imaging analysis.

The highly-oriented graphite sheet can be adhered to various types of glass slides, and therefore, are suitable for high-sensitivity, high-throughput analyses. In particular, the sheets will be an extremely useful tool for analyzing pathological sections or similar clinical samples which are normally handled in large numbers.

Another embodiment of the measurement plate for mass spectrometry according to the present invention is shown in Fig. 4. A difference from the measurement plate 10 of the first embodiment exist in that the base plate 21 is optically transparent. In the measurement plate 20 for mass spectrometry according to the present embodiment, a carbon sheet 22, which is a highly-oriented graphite sheet, is adhered to a portion of the transparent base plate 21.

On this plate 20, when a target sample 25 containing a sample to be analyzed is laid across the boundary between the carbon sheet 22 and the base plate 21, it is possible to perform a high-accuracy mass spectrometric analysis of a portion of the sample on the carbon sheet 22 along with a microscopic or similar observation of another portion of the sample having the same origin on the base plate 21. This method enables a multi-item examination using a single specimen. For example, it is possible to perform an integral analysis in which a tissue distribution is determined by an optical observation using a transmission microscope on the base plate 21 while a tissue-mass distribution is determined by a mass spectrometry on the carbon sheet 22. Specifically, when a serial section of pathological sections or similar specimen is laid across the boundary between the carbon sheet 22 and the base plate 21, both a histopathological analysis and an imaging mass analysis can be performed using the same section.

### REFERENCE SIGNS LIST

10, 20... Measurement Plate for Mass Spectrometry
11, 21... Base Plate
12, 22... Carbon Sheet
13... Electrically Conductive Adhesive (Layer)
14... High Electrical Conductivity Plate
25... Target Sample

## Claims

1. A measurement plate for MALDI mass spectrometry, comprising:
a base plate having an upper surface; and
a highly-oriented graphite sheet laid on at least a portion of the upper surface, with an orientation direction of the graphite sheet being parallel to the upper surface.

2. The measurement plate for MALDI mass spectrometry according to claim 1, wherein the highly-oriented graphite sheet has a surface roughness of 10 nm to 1 µm.

3. The measurement plate for MALDI mass spectrometry according to claim 1 or 2, wherein the base plate is electrically conductive.

4. The measurement plate for MALDI mass spectrometry according to any one of claims 1-3, wherein the base plate is made of stainless steel.

5. The measurement plate for MALDI mass spectrometry according to any one of claims 1-4, wherein the base plate and the highly-oriented graphite sheet are fixed to each other via an electrically conductive adhesive layer.

6. The measurement plate for MALDI mass spectrometry according to claim 5, wherein a high electrical conductivity plate is inserted in the electrically conductive adhesive layer.

7. The measurement plate for MALDI mass spectrometry according to any one of claims 1-3, wherein the base plate is optically transparent.

8. A method for MALDI mass spectrometry, comprising steps of:
laying a highly-oriented graphite sheet on at least a portion of an upper surface of a base plate, with an orientation direction of the graphite sheet being parallel to the upper surface; and
placing a target sample on the highly-oriented graphite sheet.

9. The method for MALDI mass spectrometry according to claim 8, wherein the highly-oriented graphite sheet has a surface roughness of 10 nm to 1 µm.

10. The method for MALDI mass spectrometry according to claim 8 or 9, wherein the base plate is electrically conductive.

11. The method for MALDI mass spectrometry according to any one of claims 8-10, wherein the base plate is made of stainless steel.

12. The method for MALDI mass spectrometry according to any one of claims 8-11, further comprising a step of fixing the base plate and the highly-oriented graphite sheet via an electrically conductive adhesive layer.

13. The method for MALDI mass spectrometry according to claim 12, further comprising a step of inserting a high electrical conductivity plate into the electrically conductive adhesive layer.

14. The method for MALDI mass spectrometry according to any one of claims 8-10, wherein the base plate is optically transparent.
